(19) Europäisches Patentamt / European Patent Office / Office européen des brevets

(11) **EP 1 658 879 B1**

(12) **EUROPEAN PATENT SPECIFICATION**

(45) Date of publication and mention of the grant of the patent:
**20.02.2013 Bulletin 2013/08**

(51) Int Cl.:
**A61P 9/10** (2006.01)

(21) Application number: **04762125.5**

(22) Date of filing: **26.08.2004**

(86) International application number:
**PCT/CN2004/000989**

(87) International publication number:
**WO 2005/049058 (02.06.2005 Gazette 2005/22)**

(54) **RADIX SALVIAE MILTIORRHIZAE, EXTRACT AND COMPOSITION THEREOF FOR THE TREATMENT OF THE ASPIRIN RESISTANCE DISEASES**

RADIX SALVIAE MILTIORRHIZAE, EXTRAKT UND ZUSAMMENSETZUNG DARAUS ZUR BEHANDLUNG VON ASPIRIN-RESISTENZ-ERKRANKUNGEN

RADIX SALVIAE MILTIORRHIZAE, EXTRAIT ET COMPOSITION DESTINES AU TRAITEMENT DES MALADIES DE RESISTANCE A L'ASPIRINE

(84) Designated Contracting States:
**AT BE BG CH CY CZ DE DK EE ES FI FR GB GR HU IE IT LI LU MC NL PL PT RO SE SI SK TR**

(30) Priority: **28.08.2003 CN 03155275**
**30.06.2004 CN 200410019838**

(43) Date of publication of application:
**24.05.2006 Bulletin 2006/21**

(73) Proprietor: **Tasly Pharmaceutical Group Co., Ltd.**
**Tianjin 300402 (CN)**

(72) Inventors:
• **YAN, Xijun**
**Beichen District,**
**Tianjin 300402 (CN)**
• **WU, Naifeng**
**Beichen District,**
**Tianjin 300402 (CN)**
• **YE, Zhengliang**
**Beichen District,**
**Tianjin 300402 (CN)**
• **LI, Xu**
**Beichen District,**
**Tianjin 300402 (CN)**
• **GUO, Zhixin**
**Beichen District,**
**Tianjin 300402 (CN)**
• **ZHAO, Na**
**Beichen District,**
**Tianjin 300402 (CN)**

(74) Representative: **Tombling, Adrian George et al**
**Withers & Rogers LLP**
**4 More London Riverside**
**London SE1 2AU (GB)**

(56) References cited:
**EP-A- 1 388 344     WO-A-02/058625**

• **ANONYMOUS: "DAN SHEN PILL" INTERNET CITATION, [Online] XP002262543 Retrieved from the Internet: URL:http://www.carbotrading.com/Products/M edicine/JZ016.htm> [retrieved on 2003-11-18]**
• **ANONYMOUS: "DAN SHEN TABLET" INTERNET CITATION, [Online] XP002262544 Retrieved from the Internet: URL:http://www.craneherb.com/products/prod uct001513> [retrieved on 2003-11-18]**
• **TENDERA M B ET AL: "Role of antiplatelet drugs in the prevention of cardiovascular events" THROMBOSIS RESEARCH 20030615 GB, vol. 110, no. 5-6, 15 June 2003 (2003-06-15), pages 355-359, XP002497031 ISSN: 0049-3848**
• **GUM PATRICIA A ET AL: "A prospective, blinded determination of the natural history of aspirin resistance among stable patients with cardiovascular disease." JOURNAL OF THE AMERICAN COLLEGE OF CARDIOLOGY 19 MAR 2003, vol. 41, no. 6, 19 March 2003 (2003-03-19), pages 961-965, XP002497032 ISSN: 0735-1097**
• **'The People's Republic of China Pharmacopoeia', part 1 2000 article 'Fufang danshen pian', pages 518 - 519**

EP 1 658 879 B1

- LIUXIAOQING ET AL.: 'The summary of fufang danshen formulations research' LISHIZHEN MEDICINE AND MATERIA MEDICA RESEARCH vol. 12, 2001, pages 474 - 475

## Description

**Technical Field**

The present invention relates to medicine. In particular, the present invention relates to the treatment of aspirin resistant cardio-cerebrovascular diseases using *Radix Salviae Miltiorrhizae* (RSM), its extract and composition comprising any or both of them, especially the formulation Danshen Drop Pills (Drop Pills of RSM).

**Background**

Aspirin belongs to a class of medicines known as non-steroidal anti-inflammatory drugs (NSAIDs). It is an effective anti-inflammatory drug with both analgesic and antipyretic effects. It works by blocking the production of prostaglandin. The most commonly known side effects of aspirin include:

- Gastrointestinal reactions, such as poor appetite, peptic ulcers, and in some cases, even perforation
- Allergic reaction
- Acute renal failure and chronic interstitial nephritis, etc.

As researches become more and more extensive, aspirin's indications have been enlarged from the treatment of fever, relieving mild to moderate pain of various kinds such as headache, toothache, neuralgia, arthralgia, myalgia and dysmenorrhea, and the treatment of rheumatism to the treatment and secondary prevention of cerebral arteriosclerosis, coronary heart disease, and myocardial infarction.

At present, aspirin is widely used in the treatment of cardiovascular disease. The mechanism is that aspirin can block the production of thromboxane A2 (TXA2) in vivo. As TXA2 can promote platelet conglutination and coagulation, aspirin can reduce the incidences of arteriosclerosis and myocardial infarction by inhibiting platelet aggregation. But researches have found that the biosynthesis of thromboxane A2 is not effectively prevented in some patients after they take the drug. That is, aspirin looses its protective effects on cerebrovascular and cardiovascular systems. This is called aspirin resistance (AR). For most patients, aspirin can reduce the risk of cardiovascular disease by 25%, but for patients with aspirin resistance, administration of aspirin, instead of protecting from cardiovascular events, can increase the incidence of myocardial infarction and stroke. These findings have limited the application of aspirin.

At present, there are not so many reports on the treatment of aspirin resistance. Yusuf S etc. reported that, in patients with acute coronary syndrome receiving aspirin, including those undergoing percutaneous coronary intervention, administration of clopidogrel in addition to treatment with aspirin is beneficial in reducing the incidence of early and long-term major cardiovascular events. ["Effects of pretreatment with clopidogrel and aspirin followed by long-term therapy in patients undergoing percutaneous coronary intervention: the PCI-CURE study". Lancet, 2001].

At present, for researches on aspirin resistance, immunoenzyme assay is commonly used for the measurement of the level of urinary 11-dehydrothromboxane $B_2$ (TXB$_2$) in the urine sample of patients before taking drug. 11-dehydrothromboxane $B_2$ is a metabolite of thromboxane $A_2$. High urinary 11-dehydrothromboxane $B_2$ level can identify patients with aspirin resistance and drugs having effects on easing aspirin resistance. It is based on this foundation that this invention is completed.

The Chinese traditional medicine for treating blood disorders, especially for activating blood circulation to dissipate blood stasis, is the commonly used medicine by doctors of all ages. This kind of medicine has many effects, such as promoting blood flow to regulate menstruation, removing blood stasis to eliminate disease, and promoting the subsidence of swelling and the regeneration of tissue. Modern pharmacological studies have confirmed that drugs used to treat blood disorders have many actions such as dilating coronary artery, increasing the coronary blood flow, reducing the oxygen consumption of cardiac muscle, reducing peripheral vascular resistance, inhibiting platelet aggregation, improving microcirculation, inhibiting thrombosis, increasing the activities of fibrinolysis, regulating anticoagulative system, lowering blood pressure, and relieving smooth muscle spasm, etc. *Rhizoma Chuanxiong,* RSM, *Herba Leonuri, Semen Persicae, Flos Carthami,* and Hirudo are among drugs used to treat blood disorders. Their clinical applications are developing continuously, and particularly the studies of RSM and its preparations are outstanding.

RSM comes from salvia, a perennial herb of salvia family. It is bitter in taste, and slightly cold in nature. It has efficacies of activating blood circulation to dissipate blood stasis, enriching blood and easing mind, cooling blood and expelling carbuncle, and expelling toxin and promoting tissue regeneration. It is a drug commonly used in traditional Chinese medicine for activating blood circulation to dissipate blood stasis. The main ingredients of RSM are liposoluble diterpenes, and water-soluble phenolic acids. In addition it contains flavonoids, triterpenes and sterols, etc. Among its diterpenes ingredients, tanshinone I , II A, II $_R$, V and VI, cryptotanshinone, isotanshinone I, II and IIB, dihydrotanshinone I and so on have a quinoid or ketoform structure. Water soluble ingredients of phenolic acids include danshensu, protocatechualdehyde, protocatechuic acid, caffeic acid, and derivatives of danshensu and caffeic acid, or depsides that are formed by esterification of dimers, such as salvianolic acid A, B, C, D, E and G, alkannic acid B, rosemary acid, methyl ester of rosemary acid, etc. Tanshinone IIA is one of the active ingredients of diterpenes for activating blood circulation to dissipate blood stasis. RSM is currently an important Chinese herbal medicine in the treatment of cardiovascular disease.

Modern researches have confirmed that RSM has pharmacological actions on:

- coronary artery
- myocardium repair and regeneration
- microcirculation
- hemorheology
- blood lipids.

These are manifested in dilating the coronary artery, anti-myocardial ischemia, anti-clotting, anti-thrombosis, sedation, alleviating pain, anti-atherosclerosis, and reducing blood lipids, etc.

The major effect of RSM on blood is the inhibition of platelet aggregation induced by adenosine diphosphate (ADP). For patients with blood stasis, the dense and sticky blood condition causes slow blood flow and the platelets tend to adhere to the endangium that has been damaged. Drugs used for activating blood circulation and dissipating blood stasis can improve hemorheology and reduce the adhesion and aggregation of platelets. Furthermore, these drugs can reduce the surface activity of platelets.

The mechanism of action of inhibition of platelet aggregation by drugs used in activating blood circulation to dissipate blood stasis is as follows: platelet aggregation is closely related to the metabolism and activity of prostaglandin and cyclic nucleotide system. Platelet thromboxane ($TXA_2$) is biosynthesized from phospholipids through many steps and using arachidonic acid as its intermediate. This process is essentially catalyzed by phosphatidase A and cyclooxygenase. The activities of these enzymes are regulated by cAMP which inhibits the activities of these enzymes and therefore the biosynthesis of $TXA_2$. If cAMP is reduced, the biosynthesis of $TXA_2$ increases. $TXA_2$ can promote the release of Ca from sarcoplasmic reticulum which is a reservoir of calcium in platelets. Ca acts on the dense granules causing adenosine diphosphate (ADP) and 5-hydroxytryptamine (5-HT) to release from them. As ADP and 5-HT are the potent promoters for platelet aggregation, the concentration of cAMP is the key factor in platelet aggregation. Increased cAMP reduces platelet aggregation. Furthermore, RSM has the activity of increasing fibrinolysis through activating the profibrinolysin-fibrinolysin system. Also, RSM can shorten the time of serum prothrombin formation.

*Radix Notoginseng* belongs to a class of traditional Chinese medicine used for hemostasia. In traditional Chinese medicine, it is regarded as having actions of dissipating blood stasis and arresting bleeding, and eliminating swelling and alleviating pain. It has the action of arresting bleeding as well as activating blood. Modern pharmacological researches have confirmed that *Radix Notoginseng* has both the hemostasis action and anti-clotting action. The hemostatic action includes slowing the bleeding and the hemoglutination progress, increasing platelets quantity and promoting the occurrence of pseudopod stretching, aggregation, and degranulation, etc. It can also reduce the permeability of blood capillary. Ingredients contained in *Radix Notoginseng* that have anti-clotting action include *Radix Notoginseng* sponin, Notoginsenoside diol and triol. They all inhibit platelet aggregation in human and rabbit. *Radix Notoginseng* sponin also promotes the secretion of tissue type profibrinolysin from the blood endothelial cells, and prevents the formation of thrombus.

Borneolum is a crystal product obtained by processing dammar, a resin obtained from plants of the dipteroarpaceae family. The crystal obtained from leaves of Blumea balsamifera Dc, a plant of the Composite family, by steam distillation, is called Praeparatio Blumeae Folii. Products synthesized and processed from camphor, terebenthene and so on through chemical methods are called Borneolum Syntheticum. Borneolum is pungent and bitter in nature. As it is aromatic, it can have a strong stimulating action on the sense organs, and dispel the stagnated fire. Its effects of inducing and promoting resuscitation are similar to Pingxiang (a Chinese medicine). The main ingredient of Borneolum is d-borneol. Praeparatio Blumeae Folii's main ingredient is 1-borneol. Modern pharmacological researches have confirmed that Borneolum has effects against myocardial ischemia, and can significantly increase coronary blood flow. Furthermore, as Borneolum can increase the permeability of blood-brain barrier, it can allow more drugs to cross the barrier.

In recent years, through clinical observations and experimental studies, the application of RSM preparations, particularly Compound Danshen Drop Pills (CDDPs, compound RSM drop pills), has further been broadened. Now it has been confirmed that it has effects against angina pectoris, and the effects of improving myocardial ischemia, reducing blood viscosity and platelet aggregation and so on. As a preparation for treatment and prevention of coronary heart disease and angina pectoris, the effect of CDDPs on the hemorheology has been confirmed. Huang Weilan etc. have carried out a comparison study on normal mice and stress stimulated mice, and found that the hemorheological indexes of the mice of both groups were improved significantly after they were given CDDPs. For example, platelet aggregation rate in 1 min and maximum aggregation rate were reduced ($P<0.05$), and content of plasmatic fibrin was reduced ($P<0.01$). These indicated a weakening of the thrombosis characteristics. In stress stimulated mice the shear rate was reduced significantly from blood viscosity under the condition of $\eta 1 \sim 100$ ($P<0.05$), indicating a process of reducing of blood circulation resistance and strengthening of circulation. Furthermore, there were observed reduction in filtration rate and enhancement in deformability of erythrocyte, but the packed cell volume was unchanged. These indicate that it is upon improving the quality of red blood cell that CDDPs achieve reduction of blood viscosity and improvement of circulation. In mice that did not undergo stimulation, although the whole blood viscosity was reduced, the reduction was insignificant (P>0.05). This means that the improvement of hemorheology by CDDPs in the abnormal hemorheological condition is more significant than in the normal hemorheological condition. Recent researches have found that CDDPs have effects of anti-atherosclerosis, lowering blood lipid, anti-fibrosis of chronic hepatopathy and so on. But up till now, there is not any reliable report about the actions of RSM preparations, particularly CDDPs, on aspirin resistance.

## Summary of the Invention

One aspect of the present invention relates to the use of RSM and its extract in the treatment of aspirin resistant cardiovascular and cerebrovascular diseases.

Or the purpose of the present invention is to provide the use of RSM and its extract in the preparation of a medicament for treating aspirin resistance.

Said aspirin resistance refers to an inability to effectively inhibit the biosynthesis of thromboxane $A_2$ after taking aspirin. That is, aspirin loses its protective effect on cardiovascular and cerebrovascular system. In the majority of patients, aspirin can reduce the risk of cardiovascular and cerebrovascular diseases by 25%. But for patients with aspirin resistance, treatment of cardiovascular and cerebrovascular diseases with aspirin can not prevent them from the cardiovascular and cerebrovascular events, but instead, can increase the risk of myocardial infarction and stroke. These findings have restricted the application of aspirin. In the present invention these cardiovascular and cerebrovascular diseases are called aspirin resistant cardiovascular and cerebrovascular diseases, particularly coronary heart disease and angina pectoris in which aspirin treatment is ineffective. In this invention, drugs that are effective in the treatment of aspirin resistant cardiovascular and cerebrovascular diseases are called drugs of anti-aspirin resistance; this action is called effect of anti-aspirin resistance.

The present invention adopts a now commonly used method for research of aspirin resistance. It uses immunoenzyme assay to test the urinary sample of the patients and analyze the change in the level of urinary 11-dehydro-thromboxane $B_2$ ($TXB_2$) to determine if there is any reduction of aspirin resistance in the patients after taking RSM preparation. From clinical investigations, the present invention confirms that RSM and its extract have effects of reducing aspirin resistance, and can be used as drugs of anti-aspirin resistance and for the preparation of drugs of anti-aspirin resistance.

Another aspect of the present invention relates to the use of a composition that contains RSM as its active ingredient in the treatment of aspirin resistant cardiovascular and cerebrovascular diseases. The compositions of the present invention include compound RSM preparations, particularly CDDPs and compound Danshen tablets (CDT, compound RSM tablets).

The following RSM compositions of this invention have preferable anti-aspirin resistance effect: RSM 30-180 parts, *Radix Notoginseng* 5-40 parts, Borneolum 0.3-2.5 parts, and adjuvants 10-40 parts. A preferred composition comprises RSM 75-115 parts, *Radix Notoginseng* 14-20 parts, Borneolum 0.8-1.2 parts, and adjuvants 15-30 parts. The most preferred composition comprises RSM 90 parts, *Radix Notoginseng* 17.6 parts, Borneolum 1 parts, and adjuvants 20 parts.

The adjuvants used in the compositions of the present invention can be any adjuvants commonly used in the art of pharmaceutics, preferably polyethylene glycol, and most preferably polyethylene glycol 6000.

RSM, its extract and compositions containing them as the active ingredients can be formulated into any pharmaceutical preparation. The preferred preparations are drop pills, spray solution, pellets, pills, granules, capsules, tablets, powder and oral liquid, etc.

For the preparation of active ingredients of the present invention, the following methods can be adopted: water extraction, water extraction combined with ethanol precipitation, extraction, impregnation, percolation, refluxing extraction, consecutive refluxing extraction, and macroporous resin adsorption. For example, these medicinal materials can be ground into powder and are mixed uniformly to form a powder preparation that can be infused orally. These medicinal materials can also be decocted with water, and then concentrated to form an oral liquid. But in order to exert the best effect of every active ingredient of the medicinal materials, it is preferable to use the following process for extraction.

RSM, 90g; *Radix Notoginseng,* 17.6g; Borneolum, 1g; polyethylene glycol 6000, 20g are provided. RSM and *Radix Notoginseng* are decocted with water for three times. The decoctions are pooled and filtrated. The filtrate is condensed. 2 volumes of 95% ethanol are added and the solution is allowed to stand still for 24 hours before filtrating. After the ethanol is recovered from the solution, the solution is condensed to reach a relative density of 1.33~1.35 (55~60°C). Borneolum is dissolved in ethanol of appropriate volume. The above-mentioned solutions of extraction and of borneolum are added into polyethylene glycol that is melted in a water bath and stirred thoroughly. The resulted solution is kept at a temperature of 70±2°C. The solution is dropped by using a dropper of appropriate diameter at a speed of 60-80 drops per minute into liquid paraffin that is cooled in an ice bath. After being shaped, the pills are taken out and mopped out the liquid paraffin from its surface with absorbent paper. 1000 pills are prepared.

Amounts of the above components can be increased or reduced proportionally when in production factory use. If it is in a large scale production, the amounts in kilograms or tons as the measure unit may be used. If it is in a small-scale production, the amounts in grams may be used. The weights of every component may be increased or reduced, but the proportions of the medicinal materials are unchanged.

The said RSM, its extract and compositions comprising them include simple recipe or compound preparations that contain RSM or its extract. These compound preparations can contain other traditional Chinese medicine or chemical drug component.

## Detailed Description of the Invention

The present invention is elaborated in details hereinafter.

## Preparation Examples

Example 1: Method for Preparing Danhong Injection (Injection of RSM and *Flos Carthami)*

1. RSM 750g, *Flos Carthami* 250g and sodium chloride for injection 7g were prepared for ready use.
2. The RSM was immersed in a diluted warm ethanol for one hour before filtrating. The extraction by immersing was repeated. The filtrates were pooled for use. The residue of RSM was combined with *Flos Carthami* before immersing in warm water for one hour followed by filtrating. The extraction by immersing was repeated. All the filtrates were pooled and condensed to a clear paste with a relative density of 1.10~1.20 (65°C). Sodium chloride for injection was added into the paste to an isotonic concentration. After the pH was adjusted to 6-7, the paste was filtrated followed by refrigerating for 24 hours. A sufficient quantity of water was added to produce the desired volume. The resulted slurry was filtrated, vialed and sterilized to produce danhong injection.

Example 2: Method for Preparing Qianglinaoxinkang Capsules

1. RSM 1500g, Armillaria Mellea extract 1500g and royal jelly 125g were prepared for ready use.
2. The RSM 75g was crushed into fine powder and the rest RSM was crushed to coarse powder. Water was added into the resulted powder to decoct three times, with the first time and the second time 2 hours and the third time 3 hours. The decoctions were combined and filtrated. The filtrate was condensed under decompression condition to a thick paste with a relative density of 1.30~1.32 (70°C). The paste was evaporated under decompression condition and crushed into fine powder. Armillaria Mellea extract was condensed under decompression condition to a thick paste having a relative density of 1.30~1.32(70°C). The paste was evaporated under decompression condition and crushed into fine powder. The fine powder was combined with the above-mentioned fine powder and mixed thoroughly. The mixture was granulated and desiccated. The royal jelly was freeze-dried to obtain fine powder. The powder was combined with the above granules and mixed thoroughly before encapsulating to produce the titled capsules.

Example 3: Method for Preparing Tongxinshu Capsules

1. Seabuckthorn flavone 250g, RSM extract 20g, *Rhizoma Chuanxiong* 10g and talcum powder 20g were prepared for ready use.
2. The *Rhizoma Chuanxiong* was crushed into fine powder and admixed with seabuckthorn flavone and RSM extract. The talcum powder was added followed by encapsulation to produce the tongxinshu capsules.

Example 4: Method for Preparing Compound Dangshen Tablets

1. Radix Codonopsis 704g, RSM 192g, Radix Angelicae Sinensis 192g, Radix Glehniae 128g, Radix Tinosporae 64g, starch 4.7g, dextrin 9.4g, talcum powder 4.3g and magnesium stearate 1.6g were prepared for ready use.

2. The Radix Tinosporae was immersed in water for 2 hours. The rest four medicinal materials including RSM were immersed in water for 1 hour. The extract thus obtained was pooled and decocted three times, each for one hour. The decoctions for three times were pooled and filtrated, followed by condensing and drying under decompression condition at a temperature less than 85°C. The dry extract resulted was crushed to coarse powder before adding starch and dextrin. The mixture was mixed up thoroughly and granulated before drying at 85°C. The granules were trimmed and the talcum powder and magnesium stearate were added before mixing thoroughly. The mixture obtained was tableted and sugar-coated to produce the desired tablets.

Example 5: Method for Preparing Danxiangguanxin Injection

1. RSM 1000g and *Lignum Dalbergiae Odoriferae* 100g were prepared for ready use.

2. The *Lignum Dalbergiae Odoriferae* was soaked with water and a sufficient quantity of water was added before distilling. About 700 ml distillate was collected and refrigerated for 24 hours. The oil layer was removed and filtrated. The aqueous layer was collected in another container. The RSM was decocted with water for three times, 2 hours for each time. The decoctions were pooled and filtrated before condensing to 500ml. The ethanol was added to obtain an ethanol content of 75% and the resulted solution was refrigerated for 48 hours before filtrating. The filtrate was condensed to 200ml by recovering the ethanol. Ethanol was added again to obtain an ethanol content of 85%. The resulted solution was refrigerated for 48 hours before filtrating. The filtrate was condensed to 120ml by recovering the ethanol. A sufficient quantity of water for injection was added to the desired volume of 1000ml and refrigerated for 16 hours before filtrating. The filtrate was condensed to a volume of 250 ml and refrigerated for 72 hours. The pH was adjusted to 6.0-6.8 with 10% solution of sodium hydroxide. The active carbon was added in an amount of 0.1%-0.4% of the medicinal materials and boiled for 30 min before filtrating. The filtrate was adjusted to pH 4 with weak hydrochloric acid. The active carbon was added again in an amount of 0.1%-0.4% of the medicinal materials before boiling 30 min. The resulted solution was refrigerated for at least 24 hours before filtrating. The filtrate was adjusted to pH 6.5-7.0 with 10% solution of sodium hydroxide. After the above distillate of *Lignum Dalbergiae Odoriferae* was added, a sufficient quantity of water for injection was added to the desired volume. The solution was filtrated, vialed and sterilized to obtain the titled injection.

Example 6: Method for Preparing Danshen injection

1. RSM 64g and glucose 50g (67g) were prepared for ready use.

2. RSM was decocted with water for three times, 2 hours for each time. The decoctions were pooled and filtrated. The filtrate was condensed to a clear paste with a relative density of 1.16 (70°C) . Ethanol was added to obtain an ethanol content of 75%. The solution was agitated thoroughly before refrigerating for 24 hours followed by filtrating. The filtrate was condensed to a clear paste with a relative density of 1.06~ 1.08 (78°C) by recovering the ethanol. The pH was adjusted to 9 with a solution of 40% sodium hydroxide. The paste was boiled for one hour before adjusting the pH to 6 with hydrochloric acid followed by filtrating. After the filtrate was cooled to room temperature, the ethanol was added to obtain an ethanol content of 85%. The solution was agitated thoroughly before refrigerating for 24 hours followed by filtrating. The filtrate was condensed to a clear paste with a relative density of 1.11 ~1.13 (78°C) by recovering the ethanol. Water for injection was added to dilute the paste 4-folds. After the pH was adjusted to 3 with hydrochloric acid, the diluted paste was refrigerated for 72 hours before filtrating. After boiling the filtrate, active carbon was added in an amount of 0.1%(g/ml). The filtrate was boiled for 15 min. before filtrating. The filtrate was held for future use. The glucose was added to the boiling water for injection to obtain a thick solution of 50%~60%. Hydrochloric acid q.s was added and active carbon was added in an amount of 0.1 % (g/ml) at the same time. The solution was agitated thoroughly, and boiled for 15 min. The solution was filtrated when it was hot to remove the active carbon. The filtrate was combined with the filtrate of RSM. The pH was adjusted to 3.8~4.2 with a solution of 10% sodium hydroxide. After boiling, active carbon was added in an amount of 0.05% (g/ml) followed by boiling for 30 min. After the solution was filtrated, a sufficient quantity of water for injection was added to the desired volume of 500ml. Sodium bisulfite 0.5g was added and mixed thoroughly. The pH was adjusted to 5~6 with the solution of 10% sodium hydroxide. Water for injection was added to the desired volume before filtrating, fine filtrating and ultrafiltrating. The filtrate was vialed and sterilized to produce the titled injection.

Example 7: Method for Preparing Jingzhi Guanxin Granules

1. RSM 350.8g, Radix Paeoniae Rubra 175.4g, *Rhizoma Chuanxiong* 175.4g, *Flos Carthami* 175.4g, *Lignum Dalbergiae Odoriferae* 116.9g, sucrose 841g and dextrin 105g were prepared for use.

2. The four medicine materials except *Flos Carthami* were decocted with water for three times, with the first time 2 hours, the second time 1.5 hours, and the last 1 hour before filtrating. The filtrates were pooled and held for future use. Immerse *Flos Carthami* was immersed in a suitable amount of warm water of 80°C for two times, with the first time 2 hours, and the second time 1 hour before filtrating. The filtrate was combined with the above filtrate and condensed to thick paste. The paste was dried at 80°C, then crushed to fine powder. The sucrose and dextrin were added and agitated thoroughly before granulating and drying to obtain the desired granulates.

Example 8: Method for Preparing Jingzhi Guanxin Tablets

1. RSM 375g, Radix Paeoniae Rubra 187.5g, *Rhizoma Chuanxiong* 187.5g, *Flos Carthami* 187.5g, *Lignum Dalbergiae Odoriferae* 187.5g, starch 12g and magnesium stearate 5g were prepared for ready use.

2. *Lignum Dalbergiae Odoriferae* was distilled to obtain volatile oil and the aqueous solution after distillation was collected in another container. The other four medicince materials including RSM were refluxed two times with 85% ethanol under heating, with the first time 3 hours and the second time 2 hours followed by filtrating. The filtrates were pooled and the ethanol was recovered. The filtrate was combined with the aqueous solution mentioned above and condensed under decompression condition to a thick paste with a relative density of 1.35-1.40 (50°C). After the starch was added, the paste was granulated by using 5# starch slurry as binder. After the granulate was dried, the volatile oil of *Lignum Dalbergiae Odoriferae* was added and agitated thoroughly. The magnesium stearate was added followed by tabletting and sugar-coating or film-coating to produce the titled tablets.

Example 9: Method for Preparing Shuxintong Capsules

1. RSM 180g, Herba Portulacae 180g, Rhizoma Homalomenae 180g, *Rhizoma Chuanxiong* 180g, *Lignum Dalbergiae Odoriferae* 200g and Borneolum 80g were prepared for ready use.

2. *Rhizoma Chuanxiong* 60g, RSM 60g, *Lignum Dalbergiae Odoriferae,* and Borneolum were crushed into fine powder respectively and held for future use. The rest *Rhizoma Chuanxiong,* RSM, Herba Portulacae and Rhizoma Homalomenae were decocted with water for two times, with the first time 1.5 hours and the second time one hour. The decoctions were filtrated and the filtrates were pooled before condensing to a clear paste with a relative density of 1.10-1.20 (90°C). Ethanol was added to obtain an ethanol content of 60% and the mixture was held for 48 hours before filtrating. The ethanol was recovered from the filtrate to obtain a thick paste with a relative density of 1.31 (80°C). The above-mentioned fine powders were added and mixed thoroughly. The mixture was dried up under at a temperature less than 80°C, and crushed into powders. The powders were mixed thoroughly and encapsulated to obtain the titled granules.

Example 10: Method for Preparing Xinnaoning Capsules

1. Folium Ginkgo 400g, Buxus microphylla 400g, Migao 400g, RSM 400g and *Bulbus Allii Macrostemi* 400g were prepared for ready use.

2. *Bulbus Allii Macrostemi* was crushed to fine powder and sieved for use. Migao was distilled with steam and the volatile oil was recovered. The residue of Migao was combined with Folium Ginkgo, Buxus microphylla, RSM and extracted with 75% ethanol. The extract was condensed under decompression condition to a clear paste by removing the ethanol. The paste was held for future use. Water was added into the residue and the mixture was decocted for 1 hour before filtrating. The filtrate was condensed to a clear paste with a relative density of 1.20 (80°C) . The paste was combined with the paste mentioned above and condensed to a thick paste. Fine powder of *Bulbus Allii Macrostemi* and the volatile oil were added into the paste and mixed thoroughly before encapsulateing to produce the titled capsulate.

Example 11: Method for Preparing Danshen Mixture

1. RSM 550g and simple syrup (65 wt.% of sucrose in water) 265ml were prepared for ready use.
2. RSM was decocted with water for two times, the first time 3 hours and the second time 2 hours. The decoctions were pooled before filtrating. The filtrate was condensed to 500ml. The ethanol was added to obtain an ethanol content of 80% and stirred thoroughly before held still for 48 hours. Supernatant was separated and the ethanol was recovered. The solution was condensed to a clear paste having a relative density of 1.12 (60-65 °C). The paste was diluted with water followed by agitating thoroughly. The resulted solution was refrigerated for 48 hours before filtrating. After the simple syrup was added into the filtrate, water was added to obtain the desired volume followed by agitating thoroughly. The solution was vialed and sterilized to produce the titled mixture.

Example 12: Method for Preparing Guanxin Danshao Tablets

1. RSM 650g, Radix Paeoniae Rubra 325g, *Rhizoma Chuanxiong* 325g, *Flos Carthami* 325g, *Lignum Dalbergiae Odoriferae* 250g and Radix Acanthopanacis Senticosi 250g were prepared for ready use.
2. 30g Radix Paeoniae Rubra was crushed into fine powder for ready use. *Lignum Dalbergiae Odoriferae* was distilled to obtain volatile oil and the aqueous solution after distillation was collected in another container. The residue of *Lignum Dalbergiae Odoriferae* and 295g Radix Paeoniae Rubra and RSM were decocted with water for two times, the first time 3 hours and the second time 2 hours. The decoctions were pooled and filtrated. The filtrate was held for future use. *Flos Carthami* was immersed in warm water (70-80°C) for two times, the first time 3 hours and the second time 2 hours, followed by filtrating. The filtrates were pooled and held for future use. *Rhizoma Chuanxiong* was extracted by refluxing using 70% ethanol for two times, the first time 8 hours and the second time 6 hours. The extracts were pooled and filtrated. The filtrate was processed by recovering the ethanol. The filtrate of *Rhizoma Chuanxiong* was combined with the above-mentioned filtrates before condensing to a thick paste with a relative density of 1.30(80°C). The above-mentioned powder was added before drying under decompression condition. The product was crushed to fine powder and sieved. The powder was mix thoroughly before granulating. After the granulates were dried, the volatile oil of *Lignum Dalbergiae Odoriferae* was sprayed over before mixing thoroughly. The mixture was tabletted and film-coated to produce the titled tablets.

Example 13: Method for Preparing Xinxinshu Capsules

1. Radix Astragali 600g, Radix Rehmanniae 360g, Fructus Schisandrae 180g, RSM 180g, Radix Paeoniae Rubra 360g, Ramulus Cinnamomi 180g and General Ginsenoside in extract of Ginseng stems and leaves 10g were prepared for ready use.
2. One third of the recipe amount of Radix Paeoniae Rubra was crushed into fine powder for ready use. The rest of Radix Paeoniae Rubra together with RSM were extracted by refluxing using ethanol for two times, each 2 hours. The extracts were pooled and filtrated. Filtrate was condensed to a thick paste with a relative density of 1.36 (60°C) by recovering the ethanol. The paste was vacuum-desiccated and crushed into fine powder for ready use. The residue of the ethanol extraction and the rest four medicines including Radix Astragali were decocted with water for three times, the first time 2 hours, the second time 1.5 hours and the third time 1 hour. The decoctions were pooled and filtrated. The filtrate was condensed to a thick paste of relative density 1.36 (60°C) under decompression condition. The fine powder of Radix Paeoniae Rubra was added and mixed thoroughly. The mixture was vacuum-desiccated and crushed into fine powder for ready use. The General Ginsenoside in extract of Ginseng stems and leaves were combined with the above two fine powders and mixed thoroughly. The mixture was granulated by using a suitable amount of ethanol followed by drying and encapsulating to obtain the titled capsules.

Example 14: Method for Preparing Danshen Tablets

1. RSM 1000g and starch 210g were prepared for ready use.
2. RSM was extracted by refluxing using 90% ethanol for 1.5 hours. The extract was filtrated and ethanol was removed from the filtrate. Water was added to the residue before decocting for one hour. The decoction was filtrated and condensed to the appropriate volume before combining with the extract by ethanol. The extract was further condensed to a thick paste with a relative density of 1.30 (90°C). The starch was added and mixed thoroughly. The mixture was desiccated, granulated, tableted and sugar-coated to obtain the titled tablets.

Example 15: Method for Preparing Xiongxiang Tongmai Pills

1. *Rhizoma Chuanxiong* 30g, Fructus Chebulae 20g, RSM 30g, Semen Myristicae 15g, Styrax 1.5g, Borneolum 0.75g, Moschus 0.15g g and polyethylene glycol 6000 15g were prepared for ready use.

2. RSM, *Rhizoma Chuanxiong,* Fructus Chebulae, and Semen Myristicae were crushed into coarse powders and extracted by using supercritical $CO_2$ respectively. The extracts were pooled and polyethylene glycol 6000 was added. Then Borneolum, Styrax and the above fine powder were added successively and mixed thoroughly. The mixture was held at 80~85°C and was dropped into pills by using methyl silicone oil as the cooling agent. Thus the titled pills were produced.

Example 16: Method for Preparing Compound Danshen Capsules

1. RSM 450g, *Radix Notoginseng* 141g, Borneolum 8g and Beta cyclodextrin 40g were prepared for ready use.

2. *Radix Notoginseng* was crushed into fine powder for future use. Borneolum was dissolved in a suitable amount of ethanol. Water was added into Beta cyclodextrin and the mixture was kept in a thermostatic water bath of 55°C to resolve Beta cyclodextrin by agitation. The mixture was stirred continuously in water bath for 30 minutes and ethanol solution of Borneolum was dropped at the same time. Then the water bath was removed and the solution was refrigerated and pump-filtrated. The residue after pump-filtration was baked at 40°C and held for future use. RSM was extracted for three times as follows. At the first time, RSM was extracted by refluxing for 1.5 hours using ethanol. The extract was filtrated and the filtrate was condensed to a thick paste with a relative density of 1.30 (555~60°C). At the second time, it was extracted by refluxing for 1.5 hours using 50% ethanol and the extract was filtrated. At the third time, it was extracted by refluxing for 2 hours using water and the extact was filtrated. The filtrates obtained at the second time and the third time were pooled and condensed to a thick paste with a relative density of 1.40 (55~60°C). The paste obtained at the first time was added and stirred thoroughly to produce a thick paste having a relative density of 1.35-1.39(55~60°C). The fine powder of *Radix Notoginseng* was added and mixed thoroughly. The obtained mixture was desiccated and crushed into fine powder. Beta cyclodextrin mixture was added and mixed thoroughly before encapsulating to produce the titled capsules.

Example 17: Method for Preparing Compound Danshen Drop Pills

1. RSM 90.0g, *Radix Notoginseng* 17.6g and Borneolum 1.0g were prepared for ready use.

2. RSM and *Radix Notoginseng* were crushed into powders and water was added in an amount of 6-folds of the medicinal materials to extract the medicinal materials for three times at a temperature between 80~90°C, the first time 3 hours, the second time 2 hours, and the third time one hour. The extracts were filtrated and the filtrates were pooled before condensing under decompression condition. Ethanol was added into the condensed filtrate to obtain an ethanol content of 55-71% so that sedimentation was resulted in. The supernatant was recovered and ethanol was removed from the supernatant before condensing to obtain a thick paste with a relative density of 1.20~ 1.25 (50~65°C). Borneolum and polyethylene glycol 6000 as much as 7-folds of the paste were added. The mixture was dropped into the liquid paraffin of 2~8°C at the temperature of 85~95°C to produce 1000 pills.

Example 18: Method for Preparing Compound Danshen Tablets (CDT)

1. RSM 450g, *Radix Notoginseng* 141 g and Borneolum 8g were prepared for ready use.

2. RSM was extracted for three times as follows. At the first time, RSM was extracted by refluxing for 1.5 hours using ethanol. The extract was filtrated and ethanol was removed from the filtrate before condensing the filtrate into a thick paste with a relative density of 1.30 (55~60°C). At the second time, it was extracted by refluxing for 1.5 hours using 50% ethanol and the extract was filtrated. At the third time, it was extracted by refluxing for 2 hours using water and the extract was filtrated. The filtrates obtained at the second time and the third time were pooled and ethanol was recovered from the filtrate. The filtrate was condensed to a thick paste with a relative density of 1.40 (55~60°C). The paste obtained at the first time was added and stirred thoroughly to produce a clear paste having a relative density of 1.35-1.39 (55~60°C). *Radix Notoginseng* was crushed into fine powders. The powders were added into the paste and mixed thoroughly. The obtained mixture was desiccated and granulated. Borneolum was crushed into fine powder and the powder was combined with the granules and mixed thoroughly. The mixture was tableted to produce 1000 tablets, which was sugar-coated or film-coated to obtain the titled tablets.

Example 19: Method for Preparing Guanxin Danshen Tablets

1. RSM 200g, *Radix Notoginseng* 200g and oil of *Lignum Dalbergiae Odoriferae* 1.75ml were prepared for ready use. 2.*Radix Notoginseng* was crushed into fine powder. RSM was crushed into medium powder and the powders were percolated using 90% ethanol as the solvent. The percolate was condensed to thick paste. The residue of the percolated material was decocted with water for two times, each one hour. The decoctions were pooled and filtrated before condensing to the desired volume. The above fine powder and thick paste were added into the condensed filtrate and stirred thoroughly. The mixture was granulated and desiccated. The oil of *Lignum Dalbergiae Odoriferae* was sprayed over the granules and mixed thoroughly. The mixture was tableted to produce 1000 tablets before sugar-coating to obtain the titled tablets.

Example 20: Method for Preparing Guanxin Danshen Drop Pills

1. RSM 200g, *Radix Notoginseng* 200g and oil of *Lignum Dalbergiae Odoriferae* 1.75ml were prepared for ready use. 2. *Radix Notoginseng* was crushed into fine powder. RSM was crushed into medium powder and the powders were percolated using 90% ethanol as the solvent. The percolate was condensed to thick paste. The residue of the percolated material was decocted with water for two times, each one hour. The decoctions were pooled and filtrated before condensing to the desired volume. The above fine powder and thick paste were added into the condensed filtrate and stirred thoroughly. An appropriate amount of polyethylene glycol was added and the oil of *Lignum Dalbergiae Odoriferae* was sprayed over before mixing thoroughly. The mixture was melted and dropped into a liquid paraffin of 0~10°C to produce 1000 the titled drop pills.

Example 21:

(a) RSM 45g, *Radix Notoginseng* 8g, Borneolum 0.5g, xylitol 12g and xanthan gum 3 g were prepared for ready use. (b) RSM and *Radix Notoginseng* were decocted with water for 5 times. The decoctions were pooled and filtrated. The filtrate was condensed before 90% ethanol in an amount of 2-folds of the condensed filtrate was added. The solution was held still for 20 hours before filtrating. Ethanol was recovered from the filtrate. The filtrate was condensed to a thick paste (1) with a relative density of 1.15~1.25(50°C). (c) Borneolum was dissolved in an appropriate amount of ethanol to obtain solution (2). Paste (1) and solution (2) were added into a mixture of xylitol and starch melted in a water bath. The mixture was stirred thoroughly and kept warmly. The mixture was dropped into methyl silicone oil at a temperature between 55~75°C and at a speed of 30~60 drops/min. to make the pills with a dropper having the caliber of 1.30~4.0mm. 1000 pills were produced. After the pills were shaped, they were taken out and mopped on surface with absorbent paper. Thus, the desired pills were obtained.

Example 22:

(a) RSM 150g, *Radix Notoginseng* 20g, Borneolum 1.5g, lactitol 83g and starch 17g were prepared for ready use. (b) RSM and *Radix Notoginseng* were decocted with water for 2 times. The decoctions were pooled and filtrated.

The filtrate was condensed before 85-95% ethanol in an amount of 4-folds of the condensed filtrate was added. The solution was held still for 36 hours before filtrating. Ethanol was recovered from the filtrate. The filtrate was condensed to a clear paste (1) with a relative density of 1.10~1.25(70-80°C).

(c) Borneolum was dissolved in an appropriate amount of ethanol to obtain solution (2). Paste (1) and solution (2) were added into a mixture of lactitol and starch melted in a water bath. The mixture was stirred thoroughly and kept warmly. The mixture was dropped into plant oil at a temperature between 65~95°C and at a speed of 20~50 drops/min. to make the pills with a dropper having the caliber of 1.10~3.0mm. 1000 pills were produced. After the pills were shaped, they were taken out and mopped on surface with absorbent paper. Thus, the desired pills were obtained.

Example 23:

(a) RSM 100g, *Radix Notoginseng* 15g, Borneolum 0.8g, xylitol 37.5g and arabic gum 12.5g were prepared for ready use.

(b) RSM and *Radix Notoginseng* were decocted with water for 3 times. The decoctions were pooled and filtrated. The filtrate was condensed before 85-90% ethanol in an amount of 4-folds of the condensed filtrate was added. The solution was held still for 18 hours before filtrating. Ethanol was recovered from the filtrate. The filtrate was condensed to a thick paste (1) with a relative density of 1.05~1.15(40-50°C).

(c) Borneolum was dissolved in an appropriate amount of ethanol to obtain solution (2). Paste (1) and solution (2) were added into a mixture of xylitol and arabic gum. The mixture was stirred thoroughly for 10 to 30 minutes and kept warmly to be melted at temperature between 50~95°C. The mixture was dropped into methyl silicone oil at a temperature between 60~85°C and at a speed of 50~60 drops/min. to make the pills with a dropper having the caliber of 1.1~3.5mm. 1000 pills were produced. After the pills were shaped, they were taken out and mopped on surface with absorbent paper. Thus, the desired pills were obtained.

Example 24:

(a) RSM 75g, *Radix Notoginseng* 14g, Borneolum 1.2g, xylitol 30.7g and arabic gum 8.3g were prepared for ready use.

(b) RSM and *Radix Notoginseng* were decocted with water for 3 times. The decoctions were pooled and filtrated. The filtrate was condensed before 95% ethanol in an amount of 3-folds of the condensed filtrate was added. The solution was held still for 24 hours before filtrating. Ethanol was recovered from the filtrate. The filtrate was condensed to a thick paste (1) with a relative density of 1.20~ 1.30(50-75°C).

(c) Borneolum was dissolved in an appropriate amount of ethanol to obtain solution (2). Paste (1) and solution (2) were added into a mixture of xylitol and arabic gum and stirred thoroughly. The mixture was melted at a temperature between 50~115°C and stirred thoroughly for 10 to 30 minutes. The mixture was kept warmly and dropped at a temperature between 60~85°C and at a speed of 20~60 drops/min. into liquid paraffin at a temperature between 0~18°C to make the pills with a dropper having the caliber of 1.1~3.5mm. 1000 pills were produced. After the pills were shaped, they were taken out and mopped on surface with absorbent paper. Thus, the desired pills were obtained.

Example 25:

(a) RSM 115g, *Radix Notoginseng* 20g, Borneolum 1.0g, xylitol 36g and pregelatine starch 4g were prepared for ready use.

(b) RSM and *Radix Notoginseng* were decocted with water for 2-4 times. The decoctions were pooled and filtrated. The filtrate was condensed before 90-97% ethanol in an amount of 1 to 3-folds of the condensed filtrate was added. The solution was held still for 18-30 hours before filtrating. Ethanol was recovered from the filtrate. The filtrate was condensed to a thick paste (1) with a relative density of 1.20~1.40(50-75°C).

(c) Borneolum was dissolved in an appropriate amount of ethanol to obtain solution (2). Paste (1) and solution (2) were added into a mixture of xylitol and pregelatine starch melted in water bath and stirred thoroughly. The mixture was melted at a temperature between 80~95°C and stirred thoroughly for 10 to 30 minutes. The mixture was kept warmly and dropped at a temperature between 60~65°C and at a speed of 40~ 60 drops/min into methyl silicone at a temperature of 0~18°C. to make the pills with a dropper having the caliber of 1.2~2.5mm. 1000 pills were produced. After the pills were dried, they were packaged. Thus, the desired pills were obtained.

Example 26:

(a) RSM 40g, *Radix Notoginseng* 17.6g, Borneolum 1g, sorbitol 15.5g and starch 4.5g were prepared for ready use.
(b) RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a thick paste (1) with a relative density of 1.20~1.40(60°C).
(c) Borneolum was dissolved in an appropriate amount of ethanol to obtain solution (2). Paste (1) and solution (2) were added into a mixture of sorbitol and starch and stirred thoroughly. The mixture was melted at a temperature between 50~75°C and stirred thoroughly for 10 to 30 minutes. The mixture was kept warmly and dropped at a temperature between 60~65°C and at a speed of 60~80 drops/min. into liquid paraffin at a temperature between 0~18°C to make the pills with a dropper having the caliber of 1.1~3.5mm, 1000 pills were produced. After the pills were shaped, they were taken out and mopped on surface with absorbent paper. Thus, the desired pills were obtained.

Example 27:

(a) RSM 90g, *Radix Notoginseng* 17.6g, Borneolum 1g, xylitol 14.6g and Carrageenan 5.4g were prepared for ready use.
(b) RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a thick paste (1) with a relative density of 1.33~1.35(55~60°C).
(c) Borneolum was dissolved in an appropriate amount of ethanol to obtain solution (2). Paste (1) and solution (2) were added into a mixture of xylitol and carrageenan and stirred thoroughly. The mixture was melted at a temperature between 80~115°C and stirred thoroughly for 10 to 30 minutes. The mixture was kept warmly and dropped at a temperature of 70±2°C and at a speed of 60~80 drops/min. into liquid plant oil at a temperature between 0~18°C to make the pills with a dropper having the caliber of 1.1~3.5mm. 1000 pills were produced. After the pills were shaped, they were taken out and mopped on surface with absorbent paper. Thus, the desired pills were obtained.

Example 28:

(a) RSM 90g, *Radix Notoginseng* 17.6g, Borneolum 1g, lactitol 16g and starch 4g were prepared for ready use.
(b) RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a thick paste (1) with a relative density of 1.33~1.35(55~60°C).
(c) Borneolum was dissolved in an appropriate amount of ethanol to obtain solution (2). Paste (1) and solution (2) were added into a mixture of lactitol and starch and stirred thoroughly. The mixture was melted at a temperature of 64°C and stirred thoroughly for 10 to 30 minutes. The mixture was kept warmly and dropped at a temperature of 64°C and at a speed of 20~60 drops/min. into methyl silicone oil at a temperature of 0°C to make the pills with a dropper having the caliber of 1.2~2.5mm. 1000 pills were produced. After the pills were shaped, they were taken out and mopped on surface with absorbent paper. After the pills were dried, they were packaged to obtain the desired pills.

Example 29:

(a) RSM 90g, *Radix Notoginseng* 17.6g, Borneolum 1g, xylitol 14g and arabic gum 6g were prepared for ready use.
(b) RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a thick paste (1) with a relative density of 1.33~1.35(55~60°C).

(c) Borneolum was dissolved in an appropriate amount of ethanol to obtain solution (2). Paste (1) and solution (2) were added into a mixture of xylitol and arabic gum and stirred thoroughly. The mixture was melted at a temperature of 64°C and stirred thoroughly for 10 to 30 minutes. The mixture was kept warmly and dropped at a temperature of 64°C and at a speed of 20~40 drops/min. into methyl silicone oil at a temperature of 10°C to make the pills with a dropper having the caliber of 1.2~2.5mm. 1000 pills were produced. After the pills were shaped, they were taken out and mopped on surface with absorbent paper. After the pills were dried, they were packaged to obtain the desired pills.

Example 30:

(a) RSM 41.06g, *Radix Notoginseng* 8.03g, Borneolum 0.46g, xylitol 12g and arabic gum 8g were prepared for ready use.

(b) RSM and *Radix Notoginseng* were crushed and decocted in an extraction pot together with 5 volumes water for 2 hours. The decoction was filtrated. The residue was decocted together with 4 volumes water for one hour to extract again. The decoction was filtrated. The filtrates were pooled and condensed under decompression condition to obtain a ratio of 1:0.9~1.1 between the volume of the condensed solution (L) and the weight of medicine materials (Kg) . 95% ethanol was added slowly to obtain an ethanol content of 69~71%. The solution was held still for 12 hours to carry on ethanol precipitation. Supernatant was collected and filtrated. Ethanol was recovered from the filtrate. The filtrate was condensed to a thick paste (1) with a relative density of 1.32~ 1.40.

(c) The paste and Borneolum were added into a mixture of xylitol and arabic gum and stirred thoroughly. The mixture was melted at a temperature of 64°C and stirred thoroughly for 10 to 30 minutes. The mixture was kept warmly and dropped at a temperature of 64°C into methyl silicone oil at a temperature of 0°C to make the pills with a dropper having the caliber of 1.2~2.5mm. The pills were taken out and mopped on surface with absorbent paper. After the pills were dried, they were packaged to obtain the desired pills.

Example 31:

(a) RSM 59.36g, *Radix Notoginseng* 6.38g, Borneolum 0.34g, lactitol 11g and arabic gum 9g were prepared for ready use.

(b) RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a thick paste (1) with a relative density of 1.33~1.35(55~60°C).

(c) Borneolum was dissolved in an appropriate amount of ethanol to obtain solution (2). Paste (1) and solution (2) were added into a mixture of lactitol and arabic gum. The mixture was melted at a temperature of 75°C and stirred thoroughly for 10 to 30 minutes. The mixture was kept warmly and dropped at a temperature of 70 °C and at a speed of 30~80 drops/min. into methyl silicone oil at a temperature of 0°C to make the pills with a dropper having the caliber of 1.2~2.5mm. 1000 pills were produced. The pills were taken out and mopped on surface with absorbent paper. After the pills were dried, they were packaged to obtain the desired pills.

Example 32:

(a) RSM 41.06g, *Radix Notoginseng* 8.03g, Borneolum 0.46g, lactitol 15g, carboxymethyl starch 3g and arabic gum 2g were prepared for ready use.

(b) RSM and *Radix Notoginseng* were crushed and decocted in an extraction pot together with 5 volumes water for 2 hours. The decoction was filtrated. The residue was decocted together with 4 volumes water for one hour to extract again. The decoction was filtrated. The filtrates were pooled and condensed under decompression condition to obtain a ratio of 1:0.9~1.1 between the volume of the condensed solution (L) and the weight of medicine materials (Kg) . 95% ethanol was added slowly to obtain an ethanol content of 69~71%. The solution was held still for 12 hours to carry on ethanol precipitation. Supernatant was collected and filtrated. Ethanol was recovered from the filtrate. The filtrate was condensed to a thick paste (1) with a relative density of 1.32~ 1.40.

(c) The paste and Borneolum were added into a mixture of lactitol, carboxymethyl starch and arabic gum in a weight ratio of 1:0.2~1:0.4 and stirred thoroughly. The mixture was melted at a temperature of 80~95°C and stirred thoroughly for 10 to 30 minutes. The mixture was kept warmly and dropped at a temperature of 60~85°C into plant oil at a temperature of 0~18°C to make the pills with a dropper having the caliber of 1.21~2.5mm. The pills were taken out, dried, and packaged to obtain the desired pills.

Example 33:

(a) The paste 7g as prepared in Example 28, Borneolum 0.1g, xylitol 18.5g and starch 1.5g were prepared for ready use.
(b) Xylitol and starch were mixed thoroughly and added into the mixture of paste and Borneolum. The mixture was melted at a temperature of 75 °C and stirred thoroughly for 10 to 30 minutes. The mixture was kept warmly and dropped at a temperature of 60~ 70°C and at a speed of 50~60 drops/min. into methyl silicone oil at a temperature of 0 °C to make the pills with a dropper having the caliber of 1.2~2.5mm. 1000 pills were produced. After the pills were shaped, they were taken out and mopped on surface with absorbent paper. The pills were dried and packaged to obtain the desired pills.

Example 34:

(a) The RSM and *Radix Notoginseng* paste 12.5g as prepared in Example 21, Borneolum 3.2g, lactitol 20g and arabic gum 3.5g were prepared for ready use.
(b) Lactitol and arabic gum were mixed thoroughly and melted at a temperature of 55~85°C. The above-mentioned paste and Borneolum were added and stirred thoroughly for 10 to 30 minutes. The mixture was kept warmly and dropped at a temperature of 60~ 75°C and at a speed of 30~50 drops/min. into plant oil at a temperature of 5~10°C to make the pills with a dropper having the caliber of 1.2~2.5mm. 1000 pills were produced. The pills were taken out and mopped on surface with absorbent paper. After the pills were dried, they were packaged to obtain the desired pills.

Example 35:

(a) The RSM and *Radix Notoginseng* paste 5.5g as prepared in Example 27, Borneolum 0.5g, xylitol 16.5g and starch 3.5g were prepared for ready use.
(b) Xylitol and starch were mixed thoroughly and melted at a temperature of 60~ 85 °C . The above-mentioned paste and Borneolum were added and stirred thoroughly for 10 to 30 minutes. The mixture was kept warmly and dropped at a temperature of 60~ 85°C and at a speed of 20~60 drops/min. into liquid paraffin at a temperature of 5~ 15°C to make the pills with a dropper having the caliber of 1.2~2.5mm. 1000 pills were produced. The pills were taken out and mopped on surface with absorbent paper. After the pills were dried, they were packaged to obtain the desired pills.

Example 36:

(a) The RSM and *Radix Notoginseng* paste 4.65g as prepared in Example 24, Borneolum 0.85g, xylitol 15.5g and arabic gum 4.5g were prepared for ready use.
(b) Xylitol and arabic gum were mixed thoroughly and melted at a temperature of 65~85°C. The above-mentioned paste and Borneolum were added and stirred thoroughly for 10 to 20 minutes. The mixture was kept warmly and dropped at a temperature of 60~ 65 °C and at a speed of 20~40 drops/min. into plant oil at a temperature of -10~15°C to make the pills with a dropper having the caliber of 1.21~2.5mm. 1000 pills were produced. The pills were taken out and mopped on surface with absorbent paper. After the pills were dried, they were packaged to obtain the desired pills.

Example 37: Preparation method for CDDP

RSM 90g, *Radix Notoginseng* 17.6g, Borneolum 1g and polyethylene glycol 6000 20g were prepared for ready use.

RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a paste (1) with a relative density of 1.33~1.35(55~60°C).

Borneolum was dissolved in an appropriate amount of ethanol to obtain solution (2). Paste (1) and solution (2) were added into polyethylene glycol melted in water bath and stirred thoroughly. The mixture was kept at a temperature of 70±2°C and dropped at a speed of 60~80 drops/min. into liquid paraffin cooled in ice bath to make the pills with a dropper having the suitable caliber. 1000 pills were produced. After the pills were shaped, they were taken out and mopped on surface with absorbent paper to obtain the desired pills.

Example 38: Preparation method for CDDP

RSM 180g, *Radix Notoginseng* 25g, Borneolum 2g and polyethylene glycol 6000 30g were prepared for ready use.

RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a paste (1) with a relative density of 1.33~1.35(55~60°C).

Borneolum was dissolved in an appropriate amount of ethanol to obtain solution (2). Paste (1) and solution (2) were added into polyethylene glycol melted in water bath and stirred thoroughly. The mixture was kept at a temperature of 70±2°C and dropped at a speed of 60~80 drops/min. into liquid paraffin cooled in ice bath to make the pills with a dropper having the suitable caliber. 1000 pills were produced. After the pills were shaped, they were taken out and mopped on surface with absorbent paper to obtain the desired pills.

Example 39: Preparation method for CDDP

RSM 115g, *Radix Notoginseng* 14g, Borneolum 1.2g and polyethylene glycol 6000 40g were prepared for ready use.

RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a paste (1) with a relative density of 1.33~1.35(55~60°C).

Borneolum was dissolved in an appropriate amount of ethanol to obtain solution (2). Paste (1) and solution (2) were added into polyethylene glycol melted in water bath and stirred thoroughly. The mixture was kept at a temperature of 70±2°C and dropped at a speed of 60~80 drops/min. into liquid paraffin cooled in ice bath to make the pills with a dropper having the suitable caliber. 1000 pills were produced. After the pills were shaped, they were taken out and mopped on surface with absorbent paper to obtain the desired pills.

Example 40: Preparation method for CDDP

RSM 30g, *Radix Notoginseng* 40g, Borneolum 0.3g and polyethylene glycol 6000 40g were prepared for ready use.

RSM and *Radix Notoginseng* were decocted with water for 3 times. The decoctions were pooled and filtrated. The filtrate was condensed before 95% ethanol in an amount of 2-folds of the condensed filtrate was added. The solution was held still for 24 hours before filtrating. Ethanol was recovered from the filtrate. The filtrate was condensed to a paste (1) with a relative density of 1.33~1.35(55~60°C).

Borneolum was dissolved in an appropriate amount of ethanol to obtain solution (2). Paste (1) and solution (2) were added into polyethylene glycol melted in water bath and stirred thoroughly. The mixture was kept at a temperature of 70±2°C and dropped at a speed of 60~80 drops/min. into liquid paraffin cooled in ice bath to make the pills with a dropper having the suitable caliber. 1000 pills were produced. After the pills were shaped, they were taken out and mopped on surface with absorbent paper to obtain the desired pills.

**Test Example 1:**

**Reduction of Aspirin Resistance by Compound Danshen Drop Pills**

Between Oct. 2003 and Jan. 2004, a screening survey of aspirin resistance for retired personnel and their couples in over 20 sanatoriums affiliated with Beijing Military Region, and identified 86 cases of aspirin resistance (56 male, 29 female, and the average age of patients: $70.9 \pm 10.9$). The incidence of aspirin resistance was about 15.6%. We divided the patients with aspirin resistance into two groups: combination therapy group (55 patients, administered aspirin and compound danshen drop pills (CDDP), referred to as "combination therapy group" hereinafter); compound danshen drop pills group (31 patients, only administered compound danshen drop pills, referred to as "CDDP group" hereinafter).

**Methods:**

1. Enrolment criteria:

- Those who incessantly administered low dose aspirin over two weeks
- Those who had the maximum platelet aggregation rate induced by arachidonic acid larger than 30% measured by the American CHRON-LOG AGGREGOMETER 540VS.

Subjects conformed with both of the above conditions were enrolled in the study.

2. Exclusion criteria:

Those having any one of the following diseases:

a) Blood system disease, particularly bleeding disease
b) Cancer
c) Chronic obstructive pulmonary diseases

Subjects conformed with any one of the above criteria should be excluded.

3. Grouping

Combination therapy group: randomly selected 55 patients with aspirin resistance administered aspirin continuously, and simultaneously administered CDDP for two weeks (30 pills/day).
CDDP group: randomly selected 31 patients with aspirin resistance administered CDDP instead of Aspirin for two weeks (30 pills/day).

4. Effect evaluation criteria

- remarkably effective: maximum platelet aggregation rate less than 30% (equal to the normal effect of aspirin)
- effective: maximum platelet aggregation rate more than 30%, but less than 80% (the rate fell in the normal range of platelet activation rate, but worse than the normal inhibition effect of aspirin)
- ineffective: variation of maximum aggregation rate before and after treatment ranged between $\pm 10\%$
- elevated: maximum aggregation rate after the treatment elevated more than 10%

$$\text{effectiveness} = \frac{(\text{number of remarkably effective patients} + \text{number of effective patients})}{\text{number of total patients}}$$

**Results**

1. Combination therapy group

(1) Effectiveness of treatment

| Treatment effect | Patients | Ratio % |
|---|---|---|
| remarkably effective | 46 | 83 |
| effective | 1 | 2 |
| ineffective | 1 | 2 |
| elevated | 7 | 13 |

The effectiveness of combination therapy group was up to 85 % (47/55).

(2) Comparison of the maximum platelet aggregation rates before and after the treatment:

t-test: Paired samples mean analysis

| | Variable 1 | Variable 2 |
|---|---|---|
| average | 69.45454545 | 27.76363636 |
| variance | 517.8821549 | 714.776431 |
| observed value | 55 | 55 |
| poisson correlation coefficient | -0.06556464 | |
| Supposed average deviation | 0 | |
| df | 54 | |
| t Stat | 8.534597132 | |
| P(T≤t) one tailed | 6.82878E-12 | |
| t one tailed critical | 1.673565748 | |
| P(T≤t) two tailed | 1.36576E-11 | |
| T two tailed critical | 2.004881026 | |

It could be seen from the above table that the platelet aggregation rate before the treatment was $69.5\pm22.8$ and it became $27.8\pm26.7$ after 2 weeks treatment (p<0.01, paired t-test) . The mean reduction of platelet aggregation rate was 51.57%.

Note：reduction＝（value before treatment－value after treatment）/ value before treatment

2. CDDP group

| (1) Effectiveness of treatment | | |
|---|---|---|
| Treatment effect | Patients | ratio % |
| remarkably effective | 12 | 39 |
| effective | 3 | 10 |
| ineffective | 5 | 16 |

(continued)

| (1) Effectiveness of treatment | | |
| --- | --- | --- |
| Treatment effect | Patients | ratio % |
| elevated | 11 | 35 |

The effectiveness of CDDP group was up to 49%(15/31)

(2). comparison of maximum platelet aggregation rates before and after the treatment

t-test: Paired samples mean analysis

| | Variable 1 | Variable 2 |
| --- | --- | --- |
| average | 69.45454545 | 27.76363636 |
| variance | 517.8821549 | 714.776431 |
| observed value | 55 | 55 |
| poisson correlation coefficient | -0.06556464 | |
| supposed average deviation | 0 | |
| df | 54 | |
| t Stat | 8.534597132 | |
| P(T≤t) one tailed | 6.82878E-12 | |
| t one tailed critical | 1.673565748 | |
| P(T≤t) two tailed | 1.36576E-11 | |
| T two tailed critical | 2.004881026 | |

It could be seen from the above table that the platelet aggregation rate before the treatment was 80.4±12.1, and it became 62.8±38.2 after 2 weeks treatment (p<0.05, paired t-test). The mean reduction of platelet aggregation rate was 18.75%.

Note：reduction＝（value before treatment－value after treatment）／ value before

treatment

Conclusion: Combination therapy of aspirin resistance by administering aspirin and CDDP simultaneously could significantly reduce platelet aggregation rate. In combination therapy group, the effectiveness was 85%, and the average reduction was 51.57%. In CDDP group, the effectiveness was 49%, and the average reduction was 18.75%. The results showed that combination therapy by administering aspirin and CDDP simultaneously could treat aspirin resistant patients who could not be treated by administering aspirin alone. Administration of CDDP could also treat aspirin resistant patient, but the effect was not so good as combination therapy. It needed further research to see whether CDDP reduces aspirin resistance by other mechanism of action.

**Test Example 2**

**Reduction of Aspirin Resistance by CDDP**

This study adopted a reviewing method, and the normal value referred to the research results about aspirin resistance in Australia.

**General Information**

All 178 patients, including 140 males and 38 females, came from the retired personnel of the First Sanatorium, Bafenbu Sanatorium. The patients were aged 70-87 and the average age was 77.1. 170 patients were randomly selected for sample test and the number of patients whose data could be accepted was 150 (IC20~IC80). Of those enrolled in the study, in addition to aspirin resistance, 91 patients were afflicted with hypertension, 25 patients were afflicted with hyperlipidemia, 32 patients were afflicted with diabetes, 139 patients were afflicted with coronary heart disease, and 57 patients were afflicted with cerebrovascular diseases. To treat aspirin resistance, 50 patients administered aspirin (aspirin group), 50 patients administered CDDP (CDDP group), and 50 patients administered both aspirin and CDDP (combination therapy group). All the patients administered drugs for at least half a year before sampling.

**Materials and methods**

The urinary sample was the middle section of the first urina of the day. The urinary sample was placed in the liquid nitrogen immediately after sampling and stored at -86°C in a low-temperature refrigerator. The urinary 11-dehydrothromboxane $B_2$ ($TXB_2$) was determined by immunoenzyme assay using the reagent kit of Cayman Chemical company. All the experiments were completed in the Biological Institute of Tasly Research Academy. Information about patient grouping was double-blind for researchers and statisticians.

**Results**

T-test was conducted for average value between groups.

Table 1. Values of urinary 11-dehydrothromboxane $B_2$ in patients of each group (ng/mmol)

| Aspirin group (n=50) | CDDP group (n=50) | Combination therapy group (n=50) |
| --- | --- | --- |
| Urinary 11-dehydro $TXB_2$ (ng/mmol) | Urinary 11-dehydro $TXB_2$ (ng/mmol) | Urinary 11-dehydro $TXB_2$ (ng/mmol) |
| 30.24 | 30.94 | 20.55 |
| 19.00 | 13.00 | 10.43 |
| 18.78 | 19.36 | 30.27 |
| 30.46 | 12.23 | 18.64 |
| 14.23 | 12.63 | 24.02 |
| 37.24 | 31.86 | 17.65 |
| 12.24 | 13.08 | 21.23 |
| 36.00 | 35.64 | 32.77 |
| 11.63 | 23.32 | 9.25 |
| 37.61 | 10.38 | 11.44 |
| 34.64 | 9.52 | 13.27 |
| 13.41 | 28.60 | 34.98 |
| 16.68 | 28.01 | 22.03 |
| 27.60 | 21.94 | 12.77 |
| 30.12 | 27.32 | 28.92 |
| 14.74 | 15.21 | 11.01 |
| 15.54 | 34.42 | 28.12 |
| 38.25 | 10.67 | 9.74 |
| 27.68 | 18.80 | 30.25 |
| 28.94 | 24.25 | 21.05 |
| 36.51 | 28.67 | 17.43 |
| 17.45 | 14.25 | 13.33 |
| 14.23 | 32.43 | 26.52 |
| 39.35 | 10.25 | 8.59 |
| 37.65 | 22.49 | 30.99 |
| 9.25 | 20.52 | 20.66 |
| 14.84 | 11.28 | 29.73 |
| 40.62 | 31.28 | 31.25 |
| 28.43 | 10.53 | 10.27 |
| 27.32 | 34.66 | 27.54 |

(continued)

| Aspirin group (n=50) | CDDP group (n=50) | Combination therapy group (n=50) |
| --- | --- | --- |
| Urinary 11-dehydro $TXB_2$ (ng/mmol) | Urinary 11-dehydro $TXB_2$ (ng/mmol) | Urinary 11-dehydro $TXB_2$ (ng/mmol) |
| 12.33 | 17.68 | 11.20 |
| 39.50 | 23.11 | 9.04 |
| 18.25 | 10.99 | 31.75 |
| 37.42 | 13.48 | 12.80 |
| 26.26 | 19.50 | 14.70 |
| 30.44 | 32.70 | 29.00 |
| 16.82 | 29.82 | 8.23 |
| 9.96 | 23.52 | 26.00 |
| 9.24 | 15.00 | 8.74 |
| 26.43 | 30.24 | 14.70 |
| 24.22 | 28.62 | 29.99 |
| 30.24 | 15.43 | 27.74 |
| 34.45 | 26.25 | 18.43 |
| 30.22 | 13.99 | 19.70 |
| 9.78 | 35.89 | 22.05 |
| 27.63 | 10.02 | 37.85 |
| 10.10 | 14.23 | 7.90 |
| 36.38 | 20.82 | 10.82 |
| 45.27 | 21.96 | 26.25 |
| 15.24 | 21.45 | 19.07 |
| X±SD 25.02±10.51 | 21.25±8.28 | 20.21±8.61 |

**Conclusion**: The excreted TXB2 between combination therapy group and aspirin group was significantly different in statistics (t=2.50 p<0.05). The excreted TXB2 between CDDP group and aspirin group was significantly different in statistics (t=1.99 p<0.05). The excreted TXB2 between combination therapy group and CDDP group was not significantly different in statistics (t=0.62 p>0.05).

The experiment data demonstrated that CDDP could reduce the excretion of urinary TXB2. That is to say, it could block the biosynthesis of thromboxane $A_2$ ($TXA_2$) *in vivo* and reduce platelet conglutination and coagulation. Accordingly, it could inhibit platelet aggregation and reduce the incidence of arteriosclerosis and myocardial infarction. The experiment results indicated that CDDP had anti-aspirin resistance effect. CDDP could improve life indexes in aspirin resistant patients with cardiovascular diseases.

**Test Example 3**

**Reduction of Aspirin Resistance by Compound Danshen Tablets**

This study used the same method as in test example 2.

150 patients in this study were all selected from the cardiovascular outpatients having the tendency of aspirin resistance. All the patients, including 80 males and 70 females, aged between 70~85, with the average age of 77. To treat aspirin resistance, 50 patients administered aspirin (aspirin group), 50 patients administered Compound Danshen Tablets (CDT group), and 50 patients administered aspirin and CDT simultaneously (combination therapy group). All the patients administered drugs for at least half a year before sampling.

**Materials and methods**

The urinary sample was the middle section of the first urina of the day. The urinary sample was placed in the liquid nitrogen immediately after sampling and stored at -86°C in a low-temperature refrigerator. The urinary 11-dehydrothromboxane $B_2$ ($TXB_2$) was determined by immunoenzyme assay using the reagent kit of Cayman Chemical company. All the experiments were completed in the Biological Institute of Tasly Research Academy. Information about patient grouping was double-blind for researchers and statisticians.

**Results**

The results were showed in table 2.

Table 2. Values of urinary 11-dehydrothromboxane $B_2$ in patients of each group (ng/mmol) $X \pm SD$

| Aspirin group (n=50) | CDT group (n=50) | Combination therapy group (n=50) |
|---|---|---|
| Urinary I 1-dehydro $TXB_2$ (ng/mmol) | Urinary 11-dehydro $TXB_2$ (ng/mmol) | Urinary I 1-dehydro $TXB_2$ (ng/mmol) |
| $25.05 \pm 10.42$ | $21.31 \pm 8.14$ | $20.53 \pm 8.23$ |

**Conclusion**: The excreted TXB2 between combination therapy group and aspirin group was significantly different in statistics ($p<0.05$). The excreted TXB2 between CDT group and aspirin group was also significantly different in statistics ($p<0.05$). The excreted TXB2 between combination therapy group and CDT group was not significantly different in statistics ($p>0.05$).

The experiment data demonstrated that CDT could reduce the excretion of urinary TXB2. That is to say, it could block the biosynthesis of thromboxane $A_2$ ($TXA_2$) *in vivo* and reduce platelet conglutination and coagulation. Accordingly, it could inhibit platelet aggregation and reduce the incidence of arteriosclerosis and myocardial infarction. The experiment results indicated that CDT had anti-aspirin resistance effect. CDT could improve life indexes in aspirin resistant patients with cardiovascular diseases.

According to the methods mentioned-above, tests were conducted on various preparations comprising RSM, such as danhong injection, qianglinaoxinkang, tongxinshu capsules, compound dangshen tablets, danxiang guanxin injection, danshen injection, jingzhi guanxin granules, jingzhi guanxin tablets, shuxintong capsules, xinnaoning capsules, guanxindanshao tablets, xinxinshu capsules, xiongxiangtongmai pills, guanxindanshen tablets, guanxindanshen drop pills etc. It was demonstrated that all the preparations comprising RSM had the effect of anti-aspirin resistance.

**Claims**

1. Use of Radix Salviae Miltiorrhizae (RSM), its extract or compositions comprising any or both of them as active ingredients in the production of medicaments for treating aspirin resistance.

2. Use of claim 1 wherein the said aspirin resistance is aspirin resistant cardio-cerebrovascular diseases.

3. Use of claim 2 wherein the said aspirin resistant cardio-cerebrovascular diseases include coronary heart disease and angina pectoris.

4. Use of claim 1 wherein the said compositions include compound preparations comprising RSM or its extract.

5. Use of claim 4 wherein the said compound preparations are selected from the group consisting of danhong injection, qianglinaoxinkang, tongxinshu capsules, compound dangshen tablets, danxiang guanxin injection, danshen injection, jingzhi guanxin granules, jingzhi guanxin tablets, shuxintong capsules, xinnaoning capsules, guanxindanshao tablets, xinxinshu capsules, xiongxiangtongmai pills, guanxindanshen tablets, guanxindanshen drop pills, compound danshen drop pills, and compound danshen tablets.

6. Use of claim 4 wherein the said compositions comprise RSM 30-180 parts, *Radix Notoginseng* 5-40 parts, Borneolum 0.3-2.5 parts, and adjuvants 10-40 parts.

7. Use of claim 6 wherein the said composition comprises RSM 75-115 parts, *Radix Notoginseng* 14-20 parts, Borneolum 0.8-1.2 parts, and adjuvants 15-30 parts.

8. Use of claim 7 wherein the said composition comprises RSM 90 parts, *Radix Notoginseng* 17.6 parts, Borneolum 1 parts, and adjuvants 20 parts.

9. Use of claim 6 wherein the adjuvants include polyethylene glycol.

10. Use of claim 4 wherein the said compositions are selected from the group consisting of drop pills, spray solution, pellets, pills, granules, capsules, tablets, powder and oral liquid.

**Patentansprüche**

1. Verwendung von Radix Salviae Miltiorrhizae (RSM), ihres Extraktes oder Zusammensetzungen, die eines oder beides umfassen, als Wirkstoffe bei der Herstellung von Medikamenten zur Behandlung von Aspirinresistenz.

2. Verwendung nach Anspruch 1, wobei die Aspirinresistenz aspirinresistente kardio- und zerebrovaskuläre Krankheiten umfasst.

3. Verwendung nach Anspruch 2, wobei die aspirinresistenten kardio- und zerebrovaskulären Krankheiten koronare Herzkrankheit und Angina pectoris einschließen.

4. Verwendung nach Anspruch 1, wobei die Zusammensetzungen RSM oder ihren Extrakt umfassende Kombinationspräparate einschließen.

5. Verwendung nach Anspruch 4, wobei die Kombinationspräparate aus der Gruppe bestehend aus Danhong-Injektion, Qianglinaoxinkang, Tongxinshu-Kapseln, zusammengesetzten Dangshen-Tabletten, Danxiangguanxin-Injektion, Danshen-Injektion, Jingzhiguanxin-Granulat, Jingzhiguanxin-Tabletten, Shuxintong-Kapseln, Xinnaoning-Kapseln, Guanxindanshao-Tabletten, Xinxinshu-Kapseln, Xiongxiangtongmai-Pillen, Guanxindanshen-Tabletten, Guanxindanshen-Tropfenpillen, zusammengesetzten Danshen-Tropfenpillen und zusammengesetzten Danshen-Tabletten ausgewählt sind.

6. Verwendung nach Anspruch 4, wobei die Zusammensetzungen 30 bis 180 Teile RSM, 5 bis 40 Teile *Radix Notoginseng,* 0,3 bis 2,5 Teile Borneolum und 10 bis 40 Teile Hilfsstoffe umfassen.

7. Verwendung nach Anspruch 6, wobei die Zusammensetzungen 75 bis 115 Teile RSM, 14 bis 20 Teile *Radix Notoginseng,* 0,8 bis 1,2 Teile Borneolum und 15-30 Teile Hilfsstoffe umfassen.

8. Verwendung nach Anspruch 7, wobei die Zusammensetzungen 90 Teile RSM, 17,6 Teile *Radix Notoginseng,* 1 Teil Borneolum und 20 Teile Hilfsstoffe umfassen.

9. Verwendung nach Anspruch 6, wobei die Hilfsstoffe Polyethylenglycol einschließen.

10. Verwendung nach Anspruch 4, wobei die Zusammensetzungen aus der Gruppe bestehend aus Tropfenpillen, Sprühlösung, Pellets, Pillen, Granulat, Kapseln, Tabletten, Pulver und oraler Flüssigkeit ausgewählt sind.

**Revendications**

1. Utilisation de *Radix Salviae Miltiorrhizae* (RSM) ou d'un extrait de RSM, ou d'une composition comprenant, en tant qu'ingrédient(s) actif(s), n'importe lequel de ces composants ou les deux, dans la production de médicaments destinés au traitement d'une résistance à l'aspirine.

2. Utilisation conforme à la revendication 1, pour laquelle ladite résistance à l'aspirine intervient dans des maladies cardio-cérébrovasculaires résistantes à l'aspirine.

3. Utilisation conforme à la revendication 2, pour laquelle lesdites maladies cardio-cérébrovasculaires englobent l'insuffisance coronarienne et l'angine de poitrine.

4. Utilisation conforme à la revendication 1, pour laquelle lesdites compositions englobent des préparations de composé(s) comprenant du RSM ou de l'extrait de RSM.

5. Utilisation conforme à la revendication 4, pour laquelle lesdites préparations de composé(s) sont choisies dans l'ensemble constitué par les suivantes : Danhong en injection, Qianglinaoxinkang, Tongxinshu en capsules, Dangshen Compound en comprimés, Danxiang Guanxin en injection, Danshen en injection, Jingzhi Guanxin en granulés, Jingzhi Guanxin en comprimés, Shuxintong en capsules, Xinnaoning en capsules, Guanxindanshao en comprimés, Xinxinshu en capsules, Xiongxiangtongmai en pilules, Guanxindanshen en comprimés, Guanxindanshen en granules, Danshen Compound en granules, et Danshen Compound en comprimés.

6. Utilisation conforme à la revendication 4, pour laquelle lesdites compositions comprennent 30 à 180 parties de RSM, 5 à 40 parties de *Radix Notoginseng,* 0,3 à 2,5 parties de Borneolum, et 10 à 40 parties d'adjuvants.

7. Utilisation conforme à la revendication 6, pour laquelle ladite composition comprend 75 à 115 parties de RSM, 14 à 20 parties de *Radix Notoginseng,* 0,8 à 1,2 partie de Borneolum, et 15 à 30 parties d'adjuvants.

8. Utilisation conforme à la revendication 7, pour laquelle ladite composition comprend 90 parties de RSM, 17,6 parties de *Radix Notoginseng,* 1 partie de Borneolum, et 20 parties d'adjuvants.

9. Utilisation conforme à la revendication 6, pour laquelle les adjuvants comprennent un polyéthylène-glycol.

10. Utilisation conforme à la revendication 4, pour laquelle lesdites compositions sont choisies dans l'ensemble constitué par les suivantes : granules, solution en pulvérisateur, pastilles, pilules, granulés, capsules, comprimés, poudre et liquide pour voie orale.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Effects of pretreatment with clopidogrel and aspirin followed by long-term therapy in patients undergoing percutaneous coronary intervention: the PCI-CURE study. *Lancet,* 2001